Europäisches Patentamt

European Patent Office

Office européen des brevets

(11) Veröffentlichungsnummer: **0 276 369 B1**

(12) **EUROPÄISCHE PATENTSCHRIFT**

(45) Veröffentlichungstag der Patentschrift: **18.12.91**

(51) Int. Cl.⁵: **A61K 31/275**

(21) Anmeldenummer: 87114495.2

(22) Anmeldetag: 05.10.87

(54) **Verwendung von Anipamil als Artereosklerosemittel.**

(30) Priorität: 09.10.86 DE 3634389

(43) Veröffentlichungstag der Anmeldung:
03.08.88 Patentblatt 88/31

(45) Bekanntmachung des Hinweises auf die
Patenterteilung:
18.12.91 Patentblatt 91/51

(84) Benannte Vertragsstaaten:
AT BE CH DE FR GB IT LI NL SE

(56) Entgegenhaltungen:
EP-A- 0 064 158
EP-A- 0 075 823

J. AM. COLL. CARDIOL., Band 1, Nr. 6, 1983,
Seiten 1453-1460, New York, US; J.-L. ROU-
LEAU et al.: "Verapamil suppresses atherosclerosis in chloresterol-fed rabbits"

AM. J. CARDIOL., Band 57, Nr. 7, Februar
1986, Seiten 1D-10D, New York, US; A. FLEK-
KENSTEIN et al.: "Antihypertensive and arterial anticalcinotic effects of calcium antagonists"

(73) Patentinhaber: KNOLL AG
Knollstrasse
W-6700 Ludwigshafen(DE)

(72) Erfinder: Lenke, Dieter, Prof. Dr.
Kekuleplatz 1
W-6700 Ludwigshafen(DE)
Erfinder: Mueller, Claus D., Dr.
Im Schaflaeger 30
W-6806 Viernheim(DE)

(74) Vertreter: Karau, Wolfgang, Dr.
BASF Aktiengesellschaft Carl-Bosch-Strasse
38
W-6700 Ludwigshafen(DE)

AM. J. CARDIOL., Band 55, Nr. 3, 1985, Seiten 165B-171B, New York, US; W.W. PARMLEY et al.: "Modification of experimental atherosclerosis by calcium-channel blockers"

BR. J. PHARMACOL., Band 88, Nr. 2, Februar 1986, Seiten 355-361, London, GB; M.J. CURTIS et al.: "Actions of the verapamil analogues, anipamil and ronipamil, against ischaemia-induced arrhythmias in conscious rats"

R. BERKOW et al.: "The Merck manual of diagnosis and therapy", 14. Ausgabe, 1982, Seiten 386-389, MSD Research Laboratories, Rahway, US

## Beschreibung

Die Erfindung betrifft eine neue Verwendung von Anipamil (= 1,7-Bis-(3-methoxyphenyl)-3-methylaza-7-cyan-nonadecan).

Anipamil ist aus der EP-A-64 158 bekannt. Dort ist angegeben, daß sich diese Verbindung zur Behandlung von funktionellen Erkrankungen des Herzkreislaufsystems, Kardiomyopathien und Angiopathien eignet. Als weitere Indikationen sind für das Anipamil hypoxische bzw. ischämische Herzerkrankungen, nichtkoronarogene Myokardschädigungen, hoher Blutdruck, Durchblutungsstörungen, Spasmen, Ulcus und allergische Reaktionen angegeben worden.

Weiter ist aus der DE-PS 1 154 810 das Verapamil (1,7-Bis-(3,4-dimethoxyphenyl)-3-methylaza-7-cyan-8-methylnonan) bekannt, welches auf seine Wirkung gegen Arteriosklerose untersucht worden ist (JACC 1, 1453 (1983), Am. J. Cardiol. 54, 884 (1984)).

Es wurde nun gefunden, daß das Anipamil eine sehr gute Wirkung gegen Arteriosklerose besitzt.

Gegenstand der Erfindung ist die Verwendung von Anipamil und dessen Salzen mit physiologisch verträglichen Säuren zur Herstellung von Arzneimitteln mit antiarteriosklerotischen Eigenschaften.

Anipamil kann in freier Form angewendet werden. Zweckmäßigerweise wird es jedoch in Form eines Salzes mit einer physiologisch verträglichen Säure (vgl. EP-A-64 158) verwendet.

Arteriosklerose ist eine häufig auftretende auf multifaktoriellen Ursachen beruhende Erkrankung der arteriellen Gefäßwand, in deren Folge es durch Bildung von Atheromen zur Einengungen der Strombahn und zu Gefäßverschlüssen kommt. Die wichtigsten für die Atherombildung verantwortlichen Faktoren sind:

- Eine Erhöhung der Permeabilität des Arterienwandendothels für Makromoleküle (Lipoproteine)
- Migration glatter Muskelzellen aus dem Verband der Gefäßmuskelschicht in die Gefäßintima mit anschließender Proliferation und Synthese von Grundsubstanzen (Bildung arteriosklerotischer Plaques)
- Einwanderung von Makrophagen welche die übermäßig eindringenden Lipoproteine aufnehmen (Schaumzellbildung).

Im weiteren Verlauf kann es zum Zerfall von Plaquegewebe und Calciumablagerung mit Mineralisation speziell der elastischen Fasern sowie zu Endothelrupturen mit Blutung in die Plaques und nachfolgender Thrombosierung des betroffenen Gefäßabschnittes (Myocardinfarkt, Schlaganfall) kommen.

Mit der erfindungsgemäßen Verbindung kann die Bildung von Atheromen in der Gefäßwand und das Fortschreiten arteriosklerotischer Veränderungen gehemmt bzw. deren Regression gefördert werden.

Zum Nachweis der antiarteriosklerotischen Wirkung wurde Anipamil männlichen Kaninchen über 10 Wochen täglich oral appliziert. Zur Erzeugung einer Arteriosklerose erhielten die Tiere eine Diät, die 2 % Cholesterin enthielt. Am Ende der Versuchsperiode wurden die Tiere getötet. Die Aorta wurde zwischen der Aortenklappe und der Bifurcatio iliaca entnommen, entlang der Medianlinie eröffnet und mit Sudan IV gefärbt. Die sich rot färbenden arteriosklerotisch veränderten Flächen der Intima wurden planimetisch bestimmt. Das Ausmaß der antiarteriosklerotischen Wirkung wurde durch Vergleich mit unbehandelten Kontrolltieren festgestellt. Als Vergleichssubstanz diente Verapamil.

In diesem Test hemmt Anipamil die Entwicklung arteriosklerotischer Plaques bereits nach Gabe von 1/4 der Substanzmenge, die zur Erzielung desselben Effekts mit Verapamil erforderlich ist.

Anipamil bzw. dessen Salze können in üblicher Weise oral oder parenteral verabfolgt werden. Die Dosierung hängt vom Alter, Zustand und Gewicht des Patienten sowie von der Applikationsart ab. In der Regel beträgt die tägliche Wirkstoffdosis zwischen etwa 0,1 und 1,0 mg/kg Körpergewicht bei oraler Gabe und zwischen 0,01 und 1,0 mg/kg Körpergewicht bei parenteraler Gabe. Im Normalfall werden tägliche Dosen von 1 bis 5 mg/kg oral oder rectal und 0,05 bis 0,25 mg/kg parenteral angewendet.

Anipamil und dessen Salze können in den gebräuchlichen galenischen Applikationsformen fest oder flüssig angewendet werden, z.B. in Form von Tabletten, Filmtabletten, Kapseln, Pulver, Granulate, Dragees, Suppositorien, Lösungen oder Dosieraerosole. Diese werden in üblicher Weise hergestellt. Die Wirkstoffe können dabei mit den gebräuchlichen galenischen Hilfsmitteln, wie Tablettenbindern, Füllstoffen, Konservierungsmitteln, Tablettensprengmitteln, Fließregulierungsmitteln, Weichmacheren, Netzmitteln, Dispergiermitteln, Emulgatoren, Lösungsmitteln, Retadierungsmitteln und/oder Antioxidantien verarbeitet werden (vgl. H. Sucker et al: Pharmazeutische Technologie, Thieme-Verlag, Stuttgart, 1978). Die so erhaltenen Zubereitungen enthalten den Wirkstoff normalerweise in einer Menge von 0,1 bis 99 Gew.%.

Beispiel 1

Auf einer Tablettenpresse werden in üblicher Weise Tabletten folgender Zusammensetzung gepreßt:

40 mg Anipamil-Hydrochlorid
120 mg Maisstärke
13,5 mg Gelatine

45 mg Milchzucker
2,25 mg Aerosil® (chemisch reine Kieselsäure in submikroskopisch feiner Verteilung)
6,75 mg Kartoffelstärke (als 6 %iger Kleister)

Beispiel 2

In üblicher Weise werden Dragees folgender Zusammensetzung hergestellt:
20 mg Anipamil-Hydrochlorid
60 mg Kernmasse
60 mg Verzuckerungsmasse

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60 : 40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 3

10 g Anipamil-Hydrochlorid werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

**Patentansprüche**

1. Verwendung von Anipamil und dessen Salzen mit physiologisch verträglichen Säuren zur Herstellung von Arzneimitteln mit antiarteriosklerotischen Eigenschaften.

**Claims**

1. Use of anipamil and its salts with physiologically tolerated acids for the preparation of drugs having anti-arteriosclerotic properties.

**Revendications**

1. Utilisation d'anipamile et ses sels avec des acides physiologiquement tolérables pour la préparation de médicaments à propriétés anti-artériosclérotiques.